# EUROPEAN PATENT SPECIFICATION

(11) **EP 3 052 130 B1**
(45) Date of publication and mention of the grant of the patent: **07.08.2019**
(21) Application number: 14850037.4
(22) Date of filing: 29.09.2014
(51) Int. Cl.: A61K 9/24, A61K 9/28, A61K 31/675, A61K 31/7068, A61P 35/00

(54) **PHARMACEUTICAL COMPOSITION COMPRISING CAPECITABINE AND CYCLOPHOSPHAMIDE**
PHARMAZEUTISCHE ZUSAMMENSETZUNG MIT CAPECITABIN UND CYCLOPHOSPHAMID
COMPOSITION PHARMACEUTIQUE COMPRENANT DE LA CAPÉCITABINE ET DU CYCLOPHOSPHAMIDE

(30) Priority: 30.09.2013 IN 3118MU2013
(43) Date of publication of application: 10.08.2016
(73) Proprietor: Intas Pharmaceuticals Limited, Ahmedabad - 380054, Gujrat (IN)
(72) Inventor: PATEL, Priyank, Ahmedabad Gujarat 380054 (IN); PATEL, Mayur, Ahmedabad Gujarat 380054 (IN); PATEL, Mahendra, Ahmedabad Gujarat 380054 (IN); SINGH, Balvir, Ahmedabad Gujarat 380054 (IN); SEHGAL Ashish, Ahmedabad Gujarat 380054 (IN)
(74) Representative: Accord Healthcare, S.L.U.
(86) International application number: PCT/IN2014/000625
(87) International publication number: WO 2015/044961

(56) References cited:
- WO-A1-2007/143212
- WO-A1-2013/028186
- WO-A2-2010/134025
- US-A- 5 047 246
- SILVIA DELLAPASQUA ET AL: "Increased mean corpuscular volume of red blood cells predicts response to metronomic capecitabine and cyclophosphamide in combination with bevacizumab", BREAST, EDINBURGH, GB, vol. 21, no. 3, 24 January 2012 (2012-01-24), pages 309-313, XP028494091, ISSN: 0960-9776, DOI: 10.1016/J.BREAST.2012.01.015 [retrieved on 2012-02-02]
- DATABASE WPI Section Ch, Week 201403 Thomson Scientific, London, GB; Class A96, AN 2013-U74552 XP002769301, WANG Y: "Capecitabine tablet composition comprises capecitabine, lactose, microcrystalline cellulose, crosslinked sodium carboxymethyl cellulose, hydroxypropyl methyl cellulose, and magnesium stearate", -& CN 103 251 569 A (CHENGDU WANDONG PHARM CO LTD) 21 August 2013 (2013-08-21)

## Description

### FIELD OF THE INVENTION

This present invention relates to pharmaceutical compositions comprising fixed dose combinations of capecitabine and cyclophosphamide, processes for the preparation thereof, and their use to treat cancer diseases.

### BACKGROUND OF THE INVENTION

Among potential active cytotoxic drugs, capecitabine is the most commonly-used agent. Capecitabine has been approved by the Food and Drug Administration in the treatment of Metastatic breast cancer (METASTATIC BREAST CANCER) patients resistant to anthracyclines and/or taxanes. Capecitabine is widely used in different combination regimens due to better therapeutic and safety profile with lower side effects. In addition, the combination partner of capecitabine plays an important role for the activation of thymidine phosphorylase (TP) enzyme, which converts the capecitabine to active 5-FU.

Cyclophosphamide is an anti-cancer chemotherapy drug. This medication is classified as an alkylating agent. Cyclophosphamide is a prodrug, converted in the liver to active forms that slow down the growth of cancer cells. Cyclophosphamide requires enzymatic and chemical activation to produce active form. Cyclophosphamide is used alone or in combination with other drugs to treat various cancers like METASTATIC BREAST CANCER, ovarian cancer, leukemia. When given orally, cyclophosphamide shows superior efficacy than when it is given intravenously.

Further interest in oral agents for the treatment of METASTATIC BREAST CANCER has increased because many patients prefer oral to intravenous regimens due to administered at home, no need to hospitalization, fear of needles, and lower associated costs. The combination of capecitabine and cyclophosphamide in present invention potentially provides an attractive, all oral alternative, giving patients more freedom and a sense of control over their treatment.

With increasing experience of capecitabine after its introduction, many clinicians found that oral administration of cyclophosphamide and capecitabine may have a greater potential for treatment of METASTATIC BREAST CANCER due to anti-angiogenesis resulting from the metronomic dosage and upregulation of thymidine phosphorylase by capecitabine. In particular, a marked reduction in the tumor volume was seen during the time period coincident with the dThdPase up-regulation. In addition, several clinical studies show that the efficacy of cyclophosphamide in combination with capecitabine was more than just additive to synergistic effects.

In view of above, there is still an existing and continual need for a fixed dose oral combination comprising capecitabine and cyclophosphamide. The inventors of the present invention address the need to provide a fixed dose combination of capecitabine and cyclophosphamide to allow convenient administration of both drugs as a single tablet and to effective treatment with good acceptability at lower dose.

WO 2013/028186 generally discloses a method of treating cancer comprising administering to the patient a single oral composition comprising a combination of at least two chemotherapeutic agents such as capecitabine and cyclophosphamide, optionally comprising additional therapeutic or chemotherapeutic agents, e.g., anti-emetics or cytotoxic compounds.

Inventors of the present invention investigated the development of a pharmaceutical composition comprising capecitabine and cyclophosphamide for oral administration.

However, a stable pharmaceutical composition was not obtained due to incompatibility between the two therapeutic agents, specifically total impurity levels were found to be increased drastically.

To overcome the issue related to incompatibilities of both therapeutic agents and pharmaceutical excipients, the inventors have provided herewith a novel stable pharmaceutical composition which can be formulated for oral administration.

Furthermore, the present invention provides the advantages of combination therapy while reducing the number of prescriptions, number of tablets to be taken which results in better patient compliance and attendant administrative costs. Combination therapies with agents having complementary mechanisms of action may provide advantages of each type of agent and reduce some of the adverse effects of high-dose of individual drugs.

### OBJECTS OF THE INVENTION

The object of the present invention is to provide a solid pharmaceutical composition for oral administration comprising a fixed dose combination of capecitabine and cyclophosphamide in the form of a bilayer tablet, wherein the bilayer tablet comprises a first layer and a second layer, wherein the first layer comprises capecitabine and one or more excipients, and the second layer comprises cyclophosphamide and one or more excipients, and further optionally comprising a film coating that covers both layers.

Another object of the present invention is to provide a process for the preparation of the above pharmaceutical composition comprising a first layer and a second layer, wherein the first layer comprises capecitabine and one or more excipients, and the second layer comprises cyclophosphamide and one or more excipients, and further optionally comprising a film coating that covers both layers.

Another object of the invention is to provide the above pharmaceutical composition for use in treating metastatic breast cancer.

The scope of the invention is defined by the claims. Any references in the description to methods of treatment refer to the compounds, pharmaceutical compositions and medicaments of the present invention for use in a method of treatment of the human (or animal) body by therapy.

### SUMMARY OF THE INVENTION

The present invention relates to pharmaceutical compositions comprising fixed dose combinations of capecitabine and cyclophosphamide in solid dosage form for oral administration, processes for the preparation thereof, and their use to treat cancer diseases. Further the combination of capecitabine and cyclophosphamide is an effective, convenient and well-tolerated regimen for Metastatic Breast Cancer.

### DETAILED DESCRIPTION

Unless otherwise indicated, terms in this specification are intended to have their ordinary meaning in the relevant art.

The present invention relates to a fixed dose combination comprising capecitabine and cyclophosphamide in the form of a bilayer tablet. Oral combination of capecitabine and cyclophosphamide are conventional drugs for the treatment and are an effective and well-tolerated regimen for Metastatic Breast Cancer.

According to present invention the pharmaceutical composition comprising a fixed dose combination present in solid dosage form, particularly in oral form, is a bilayer tablet.

The fixed dose combination according to present invention is provided in the form of a pharmaceutical bilayer tablet composition comprising a first layer and a second layer, wherein the first layer comprises capecitabine and one or more excipients, and the second layer comprises cyclophosphamide and one or more excipients.

According to present invention the bilayer tablet further optionally comprises a film coating that covers both layers.

In general, excipients which may be used may typically be selected from the group consisting of one or more diluents or fillers, one or more binders, one or more glidants, one or more disintegrants, one or more lubricants, and the like. The amount of each excipient in a solid dosage formulation may vary within ranges conventional in the art.

The pharmaceutical composition described herein may be prepared by conventional technology well known to those skilled in the art such as wet granulation, dry granulation and direct compression and the like.

More preferably in the present invention, the first layer comprising capecitabine can be prepared by wet granulation as hereinafter described whilst the second layer comprising cyclophosphamide can be prepared by blending the excipients for direct compression.

Alternatively, the second layer comprising cyclophosphamide can be prepared by wet granulation. Both layers can then be combined and compressed together as herein after described. Furthermore, the bilayer tablet dosage form may comprise a film coating. Suitable film coating is known and commercially available or can be made according to known methods.

According to present invention the film coating material is a polymeric film coating material comprising hydroxypropylmethyl cellulose, polyethylene glycol, polysorbate, sodium carboxy methyl cellulose, Talc, Titanium dioxide, simethicon, Eudragit, purified water and colorant.

According to one of the embodiments, a bilayer tablet according to present invention generally contains 50-1800 mg, preferably 100-1500 mg, more preferably 300-800 mg capecitabine; and 10-100 mg, preferably 20-80 mg, more preferably 20-60mg cyclophosphamide. Presently preferred forms are bilayer tablet comprising 300/20 mg, 400/20 mg, 600/40 and 700/30 mg of capecitabine and cyclophosphamide, respectively.

The first tablet layer according to present invention comprises capecitabine as active agent and one or more excipients. The capecitabine containing first layer of the invention is prepared by wet granulation. Alternative methods for granulation of the active ingredient and excipients with a granulation liquid are fluid bed granulation or top spray granulation.

In the wet granulation process the granulating liquid is a solvent such as purified water, ethanol, isopropanol, acetone or mixture thereof, preferably purified water. The solvent is a volatile component, which does not remain in the final product.

Excipients of the first layer may be particularly selected from the group consisting of one or more fillers, one or more binders, one or more disintegrants, and one or more lubricants.

According to present invention the first layer of the bilayer tablet is prepared by wet granulation comprising following steps:
a) sifting of capecitabine, one or more filler, one or more disintegrant through appropriate mesh and mixing in a suitable mixer,
b) dissolving a binder in a solvent to produce a granulation liquid,
c) carrying out fluid bed granulation using the granulating liquid of step (b) for spraying onto the mixture of step (a),
d) after completion of the granulation drying and optionally screening the granulate obtained in step (c),
e) optionally blending the granulate obtained in step (d) with additional excipients; and
f) lubricating the blended granules obtained in step (e) to prepare the final composition of first layer.

Alternatively, a binder can be added with the blend obtained in step (a) and further granulation is done with a suitable solvent which would act as a granulation liquid.

Examples of filler for first layer include, without being limited to, microcrystalline cellulose, mannitol, sucrose or other sugar or sugar derivatives, low substituted HPC, dicalcium phosphate, lactose and combination thereof.

Examples of binder for first layer include, without being limited to, polyvinylpyrrolidone, hydroxypropyl methylcellulose, hydroxypropyl cellulose, pregelatinized starch, maize starch, microcrystalline cellulose (e.g., cellulose MK GR), and combinations thereof.

Examples of disintegrant for first layer include, without being limited to, croscarmelose sodium, crospovidone, sodium starch glycolate, starch, pregelatinized starch and combination thereof.

Examples of lubricant for first layer include, without being limited to, magnesium stearate, calcium stearate, aluminum or calcium silicate, stearic acid, talc and combinations thereof.

The second tablet layer according to present invention comprises cyclophosphamide as active agent and one or more excipients.

According to one of the embodiments, the second layer comprises cyclophosphamide having a D90 particle size of less than 300 microns, more preferably 100 microns.

According to present invention the second layer of the bilayer tablet is prepared by direct compression comprising following steps:
a) mixing the cyclophosphamide with one or more filler, one or more binder, one or more disintegrant in a suitable mixer,
b) adding one or more lubricant in mixture obtained in step (a) and blending them until obtaining a homogenous powder mixture; and
c) compressing the final powder mixture to form final composition of second layer.

According to present invention the second layer of the bilayer tablet can also be prepared by wet granulation comprising following steps:
(a) Co-sift one or more pharmaceutically acceptable excipients and prepare a mixture,
(b) granulate the above mixture by using solvent,
(c) dry the above granulate and mill the dried granules,
(d) prepare the drug mixture separately by co-sifting cyclophosphamide and one or more pharmaceutically acceptable excipients,
(e) blend the granules obtained in step (c) and drug mixture of step (d); and
(f) lubricate the blended mixture obtained in step (e) to form final blend for second layer.

Excipients of the second layer may be particularly selected from the group consisting of one or more fillers, one or more binders, one or more disintegrants, and one or more lubricants.

Examples of filler for second layer include, without being limited to, dibasic calcium phosphate anhydrous, microcrystalline cellulose, lactose, mannitol, sucrose or other sugar or sugar derivatives, low substituted HPC, pregelatinized starch, and combination thereof.

Examples of binder for second layer include, without being limited to, polyvinylpyrrolidone, povidone, copovidone, hydroxypropyl methylcellulose, hydroxypropyl cellulose, pregelatinized starch, maize starch, microcrystalline cellulose (e.g., cellulose MK GR), and combinations thereof.

Examples of disintegrant for second layer include, without being limited to, croscarmelose sodium, crospovidone, sodium starch glycolate, starch, pregelatinized starch and combination thereof.

Examples of lubricant for second layer include, without being limited to, magnesium stearate, calcium stearate, aluminum or calcium silicate, stearic acid, talc and combinations thereof.

For preparing a bilayer tablet according to present invention, the first and second tablet layers prepared as described hereinabove may be compressed in the usual manner in a bilayer tablet press.

Another preferred aspect of the present invention also includes an optional film coating on the bilayer tablet. The details regarding the film coating material component are as described herein above.

According to present invention capecitabine and cyclophosphamide are physically incompatible substances. When both drugs were kept on 40°C (open) for 1 month, different type of impurities related with cyclophosphamide are obtained during preformulation studies. The % impurities related with cyclophosphamide obtained during the preformulation studies are as below.

**Table 1: Pre-formulation studies related to combination of Capecitabine + cyclophosphamide**

| **Sample** | **Time period** | **Impurity A*** | **Impurity B*** | **Impurity C*** | **Impurity D*** | **Total Impurity** |
|---|---|---|---|---|---|---|
| Capecitabine + Cyclophosphamide | Initial | ND | ND | ND | 0.053% | 0.234% |
| | 1 month | ND | ND | 0.296% | 13.758% | 16.992% |
| [Capecitabine + Cyclophosphamide] + MCC PH101 | Initial | ND | ND | ND | 0.033% | 0.865% |
| | 1 month | ND | ND | 0.235% | 12.407% | 14.499% |
| [Capecitabine + Cyclophosphamide] + Lactose | Initial | ND | ND | ND | 0.025% | 0.204% |
| | 1 month | ND | ND | 0.113% | 8.178% | 10.146% |
| [Capecitabine + Cyclophosphamide] + Croscarmellose Sodium | Initial | ND | ND | ND | 0.575% | 1.010% |
| | 1 month | ND | ND | 0.086% | 5.014% | 5.893% |
| [Capecitabine + Cyclophosphamide] + Magnesium Stearate | Initial | ND | ND | ND | 0.022% | 0.192% |
| | 1 month | ND | ND | 0.091% | 6.658% | 8.220% |

| | | | | | | |
|---|---|---|---|---|---|---|
| *Impurity A of cyclophosphamide is chemically 3-[3-(2-chloroethylamino) ethyl amino] propyl dihydrogen phosphate. *Impurity B of cyclophosphamide is 3-aminopropyl dihydrogen phosphate. *Impurity C of cyclophosphamide is 3-3 chloroethyl-2-oxo-2-hydroxy-1, 3, 6, 2 oxadiazaphosphonane. *Impurity D of cyclophosphamide is Bis (2-chloroethyl)amine hydrochloride. | | | | | | |

Acceptable limits of the above said impurities of cyclophosphamide according to the present invention are individually not more than 0.5% w/w and the total impurity of cyclophosphamide should not be more than 3% w/w, when determined after one month when kept at 40°C.

Accordingly, the pre-formulation studies for combination of capecitabine and cyclophosphamide do not comply with the above said acceptable limits. Further the present invention provides a pharmaceutical composition comprising a fixed dose combination of capecitabine and cyclophosphamide which has a greater potential for treatment of metastatic breast cancer.

In addition, the present invention provides a better therapeutic efficacy by combined administration of capecitabine and cyclophosphamide rather than when used separately.

### EXAMPLES

The scope of the invention is in no manner limited by the disclosed examples.

### Example 1 and 2

| **Ingredients** | **Example 1 Mg/tab** | **Example 2 Mg / Tab** |
|---|---|---|
| Capecitabine | 300.0 | 600.0 |
| Microcrystalline Cellulose (Avicel PH 112) | 57.0 | 114.0 |
| Croscarmellose Sodium | 16.0 | 32.0 |
| HPMC E-5 | 15.0 | 30.0 |
| Purified Water | q.s. | q.s. |
| Croscarmellose Sodium | 4.0 | 8.0 |
| Magnesium Stearate | 8.0 | 16.0 |
| **Total of Layer I** | **400.00** | **800.00** |
| Cyclophosphamide | 21.40 | 42.80 |
| Microcrystalline Cellulose (Avicel PH 112) | 133.80 | 267.60 |
| Povidone K-90 | 3.40 | 6.80 |
| Croscarmellose Sodium | 8.00 | 16.00 |
| Magnesium Stearate | 3.40 | 6.80 |
| **Total Layer II** | **170.0** | **340.0** |
| **Total Core Tablet weight** | **570.00** | **1140.00** |
| Polyethylene glycol 4000 | 1.96 | 3.92 |
| Polysorbate 80 | 0.40 | 0.80 |
| Sodium carboxymethylcellulose | 0.32 | 0.64 |
| Talc | 3.09 | 6.18 |
| Titanium Dioxide | 3.09 | 6.18 |
| Eudragit NE30D | 2.94 | 5.88 |
| Ferric oxide Red | 0.1 | 0.2 |
| Ferric oxide yellow | 0.1 | 0.2 |
| Purified water | q.s. | q.s. |
| **Total coated tablet weight** | **582.00** | **1164.00** |

### Brief Manufacturing process of Example 1 and 2

### Preparation of Capecitabine Layer:

1. Sift capecitabine, microcrystalline cellulose (Avicel PH112), croscarmellose sodium, through ASTM 20# sieve.
2. Place materials of step 1 in fluid bed energizer and dry mix for 5 min at 50°C.
3. Dissolve hypromellose E5 in Purified Water using stirrer.
4. Granulate materials in fluid bed energizer using binder solution of step 3.
5. Dry the granules in fluid bed energizer at 55°C.
6. Pass the dried granules through ASTM 20# sieve.
7. Sift croscarmellose sodium through ASTM 20# sieve and mix with granules of step 6 in blender for 10 mins at 25 RPM.
8. Sift magnesium stearate through ASTM 40# sieve and mix with blend of step 7 for 3 mins.

### Preparation of Cyclophosphamide Layer:

9. Separately, sift cyclophosphamide, microcrystalline cellulose (Avicel PH 112), povidone k-90 and croscarmellose sodium, through ASTM 20# sieve. Mix it in blender for 10 mins at 25 RPM.
10. Sift magnesium stearate through 40# sieve and mix with blend of step 9 for 3 mins at 25 RPM.

### Compression of Bilayer tablet

11. Bilayer tablets were compressed using blend of step 8 and blend of step 10 using rotary tablet compression machine.
12. Tablets were coated using coating solution containing polyethylene glycol 6000, polysorbate 80, sodium carboxymethyl cellulose, talc, titanium dioxide, eudragit NE30D, ferric oxide red, ferric oxide yellow and purified water.
13. Pack the film coated tablets in suitable pack using packaging machine.

### Example 3 and 4

| **Ingredients** | **Example 3 (mg / tab)** | **Example 4 (mg / tab)** |
|---|---|---|
| Capecitabine | 400.0 | 700.0 |
| Microcrystalline Cellulose (Avicel PH 101) | 33.27 | 58.22 |
| Lactose anhydrous | 38.43 | 60.72 |
| Croscarmellose Sodium (Ac-di-sol) | 13.25 | 23.19 |
| HPMC E-5 | 18.55 | 32.46 |
| Purified Water | q.s. | q.s. |
| Croscarmellose Sodium (Ac-di-sol) | 13.25 | 23.19 |
| Colloidal silicon dioxide (Aerosil-200) | 2.65 | 4.64 |
| Magnesium Stearate | 10.60 | 18.55 |
| **Total of Layer I** | **530.00** | **930.00** |
| Microcrystalline cellulose (Avicel PH 101) | 62.550 | 93.83 |
| Dibasic calcium phosphate, dihydrate (milled) | 38.400 | 57.60 |
| Pregelatinized starch (Starch 1500) | 21.000 | 31.50 |
| Povidone K-90 | 3.40 | 5.10 |
| Purified water | q.s. | q.s. |
| Cyclophosphamide | 24.40 | 32.1 |
| Pregelatinized starch (Starch 1500) | 4.60 | 6.90 |
| Croscarmellose Sodium (Ac-di-sol) | 10.00 | 15.00 |
| Talc | 3.40 | 5.10 |
| Colloidal anhydrous silica-E | 1.70 | 2.55 |
| Magnesium Stearate | 3.40 | 5.10 |
| Ferric oxide yellow | 0.150 | 0.23 |
| **Total Layer II** | **170.0** | **255.0** |
| **Total Core Tablet weight** | **700.00** | **1185.00** |
| Polyethylene glycol 4000 | 2.39 | 4.78 |
| Polysorbate 80 | 0.49 | 0.98 |
| Sodium carboxymethylcellulose | 0.39 | 0.78 |
| Talc | 4.74 | 9.48 |
| Titanium Dioxide | 4.74 | 9.48 |
| Eudragit NE30D | 2.152 | 4.30 |
| Ferric oxide yellow | 0.09 | 0.18 |
| Ferric oxide Red | 0.004 | 0.01 |
| Purified water | q.s. | q.s. |
| **Total coated tablet weight** | **715.00** | **1215.00** |

### Brief Manufacturing process of Example 3 and 4

### Preparation of Capecitabine Layer:

Process for the preparation of capecitabine layer was similar as per example 1 and 2.

### Preparation of Cyclophosphamide Layer:

9. Co-sift microcrystalline cellulose, Dibasic calcium phosphate dehydrate, Pregelatinized starch and Povidone K-90 through 30#ASTM sieve.
10. Dry mix and granulate the blend of step 9 using Purified water. Dry the granules at 60°C. Mill the granules through co mill.
11. Separately, co-sift cyclophosphamide, pregelatinized starch (Starch 1500) and croscarmellose sodium through 40# ASTM sieve.
12. Co-sift talc and colloidal anhydrous silica-E through 40#ASTM sieve. Ferric oxide yellow was sifted through 80# ASTM sieve.
13. Blend the granules of step 10, 11 and 12 in blender.
14. Sift magnesium stearate through 40# sieve and mix with blend of step 13.

### Compression and Film coating of Bilayer tablet

Process for preparation of film coating is similar as example 1 and 2.

### In-vitro dissolution profile

The bilayer tablets of fixed dose combination of capecitabine and cyclophosphamide prepared as per the compositions of example 1 to example 4 were subjected to dissolution studies in 900 ml of phosphate buffer pH 6.8 at 37±0.5°C using basket apparatus with rotational speed at 100 rpm.

Table 2 provides dissolution profile of tablets prepared according to example 1 to example 4.

| **Time (Min)** | **% Drug Release** | | | |
|---|---|---|---|---|
| | **Example 1** | **Example 2** | **Example 3** | **Example 4** |
| 5 | 7 | 6 | 10 | 09 |
| 10 | 24 | 15 | 38 | 23 |
| 15 | 45 | 26 | 52 | 40 |
| 20 | 69 | 40 | 81 | 63 |
| 30 | 94 | 73 | 99 | 91 |
| 45 | 100 | 93 | 103 | 101 |
| 60 | 100 | 97 | 103 | 103 |

The above dissolution study data comply with the dissolution testing requirements of immediate release solid oral dosage forms.

### Stability study

The study of cyclophosphamide impurities A, B, C, and D profile of example 1 to 4 was carried out at 30°C / 65% RH for 1 month.

The impurity profile results obtained are as below:

| | **Time period** | **Impurity A** | **Impurity B** | **Impurity C** | **Impurity D** | **Total Impurity** |
|---|---|---|---|---|---|---|
| **Example 1** | Initial | ND | ND | ND | ND | ND |
| | 1 months | ND | ND | ND | ND | 0.023 |
| **Example 2** | Initial | ND | ND | ND | ND | ND |
| | 1 months | 0.033% | ND | ND | 0.045% | 0.078% |
| **Example 3** | Initial | ND | ND | ND | ND | 0.085% |
| | 1 months | ND | 0.072% | ND | 0.075% | 0.308% |
| **Example 4** | Initial | ND | ND | ND | ND | 0.106% |
| | 1 months | ND | 0.058% | ND | 0.025% | 0.241% |

Impurity profile of the pharmaceutical compositions according to examples 1 to 4 meets the acceptance criteria of individual and total impurities of cyclophosphamide as disclosed hereinabove.

## Claims

1. A solid oral pharmaceutical composition in the form of a bilayer tablet comprising a fixed dose combination of capecitabine and cyclophosphamide, wherein the bilayer tablet comprises a first layer and a second layer wherein the first layer comprises capecitabine and one or more pharmaceutically acceptable excipients and the second layer comprises cyclophosphamide and one or more pharmaceutically acceptable excipients and further optionally comprising a film coating that covers both layers.

2. The solid oral pharmaceutical composition according to claim 1, wherein the first layer contains 100 to 1500 mg of capecitabine.

3. The solid oral pharmaceutical composition according to claims 1 and 2, wherein the second layer contains 10 to 100 mg of cyclophosphamide.

4. The solid oral pharmaceutical composition according to any preceding claim, wherein the pharmaceutically acceptable excipients can be selected from one or more diluents or fillers, one or more binders, one or more glidants, one or more disintegrants, one or more lubricants and combinations thereof.

5. A process for the preparation of a solid oral pharmaceutical composition according to claim 1 comprising a first layer and a second layer wherein the first layer comprises capecitabine and one or more pharmaceutically acceptable excipients and the second layer comprises cyclophosphamide and one or more pharmaceutically acceptable excipients and further optionally comprising a film coating that covers both layers.

6. The solid oral pharmaceutical composition of claim 1 for use in treating metastatic breast cancer.

## Patentansprüche

1. Feste orale pharmazeutische Zusammensetzung in Form einer Zweischichttablette, umfassend eine Kombination von Capecitabin und Cyclophosphamid in fixer Dosis, wobei die Zweischichttablette eine erste Schicht und eine zweite Schicht, wobei die erste Schicht Capecitabin und einen oder mehrere pharmazeutisch unbedenkliche Exzipienten umfasst und die zweite Schicht Cyclophosphamid und einen oder mehrere pharmazeutisch unbedenkliche Exzipienten umfasst, und gegebenenfalls weiterhin einen die beiden Schichten abdeckenden Filmüberzug umfasst.

2. Feste orale pharmazeutische Zusammensetzung nach Anspruch 1, wobei die erste Schicht 100 bis 1500 mg Capecitabin enthält.

3. Feste orale pharmazeutische Zusammensetzung nach Anspruch 1 oder 2, wobei die zweite Schicht 10 bis 100 mg Cyclophosphamid enthält.

4. Feste orale pharmazeutische Zusammensetzung nach einem der vorhergehenden Ansprüche, wobei die pharmazeutisch unbedenklichen Exzipienten aus einem oder mehreren Verdünnungsmitteln oder Füllstoffen, einem oder mehreren Bindemitteln, einem oder mehreren Gleitmitteln, einem oder mehreren Sprengmitteln, einem oder mehreren Schmiermitteln und Kombinationen davon ausgewählt sein können.

5. Verfahren zur Herstellung einer festen oralen pharmazeutischen Zusammensetzung nach Anspruch 1, umfassend eine erste Schicht und eine zweite Schicht, wobei die erste Schicht Capecitabin und einen oder mehrere pharmazeutisch unbedenkliche Exzipienten umfasst und die zweite Schicht Cyclophosphamid und einen oder mehrere pharmazeutisch unbedenkliche Exzipienten umfasst, und weiterhin gegebenenfalls umfassend einen die beiden Schichten abdeckenden Filmüberzug.

6. Feste orale pharmazeutische Zusammensetzung nach Anspruch 1 zur Verwendung bei der Behandlung von metastasiertem Brustkrebs.

## Revendications

1. Composition pharmaceutique orale solide sous la forme d'un comprimé bicouche comprenant une combinaison à dose fixe de capécitabine et de cyclophosphamide, où le comprimé bicouche comprend une première couche et une deuxième couche, où la première couche comprend de la capécitabine et un ou plusieurs excipients pharmaceutiquement acceptables et la deuxième couche comprend du cyclophosphamide et un ou plusieurs excipients pharmaceutiquement acceptables et en outre, éventuellement, une pellicule d'enrobage recouvrant les deux couches.

2. Composition pharmaceutique orale solide selon la revendication 1, où la première couche comprend 100 à 1500 mg de capécitabine.

3. Composition pharmaceutique orale solide selon les revendications 1 et 2, où la deuxième couche comprend 10 à 100 mg de cyclophosphamide.

4. Composition pharmaceutique orale solide selon l'une quelconque des revendications précédentes, où les excipients pharmaceutiquement acceptables peuvent être choisis parmi un ou plusieurs diluants ou charges, un ou plusieurs agents liants, un ou plusieurs agents glissants, un ou plusieurs agents délitants, un ou plusieurs lubrifiants et leurs combinaisons.

5. Procédé de préparation d'une composition pharmaceutique orale solide selon la revendication 1 comprenant une première couche et une deuxième couche, où la première couche comprend de la capécitabine et un ou plusieurs excipients pharmaceutiquement acceptables et la deuxième couche comprend du cyclophosphamide et un ou plusieurs excipients pharmaceutiquement acceptables et en outre, éventuellement, une pellicule d'enrobage qui recouvre les deux couches.

6. Composition pharmaceutique orale solide selon la revendication 1 pour utilisation dans le traitement d'un cancer du sein métastatique.
